(19) Europäisches Patentamt
European Patent Office
Office européen des brevets

(11) Veröffentlichungsnummer : **0 129 748**
**B1**

(12) # EUROPÄISCHE PATENTSCHRIFT

(45) Veröffentlichungstag der Patentschrift : **30.09.87**

(21) Anmeldenummer : **84106420.7**

(22) Anmeldetag : **05.06.84**

(51) Int. Cl.[4] : **C 07 D305/12** // (C07D305/12, C12P17:02, C12R1:465)

(54) **Hexadecansäure- und Hexadecadiensäurederivate.**

(30) Priorität : **22.06.83 CH 3415/83**

(43) Veröffentlichungstag der Anmeldung : **02.01.85 Patentblatt 85/01**

(45) Bekanntmachung des Hinweises auf die Patenterteilung : **30.09.87 Patentblatt 87/40**

(84) Benannte Vertragsstaaten : **AT BE CH DE FR GB IT LI LU NL SE**

(56) Entgegenhaltungen :
**FR-A- 2 379 531**
**FR-A- 2 426 679**

(73) Patentinhaber : **F. HOFFMANN-LA ROCHE & CO. Aktiengesellschaft**
**CH-4002 Basel (CH)**

(72) Erfinder : **Hadvary, Paul, Dr.**
**Neumattenweg 8**
**CH-4105 Biel-Benken (CH)**
Erfinder : **Hochuli, Erich, Dr.**
**Kirchackerstrasse 25**
**CH-4411 Arisdorf (CH)**
Erfinder : **Kupfer, Ernst, Dr.**
**St. Alban-Rheinweg 180**
**CH-4052 Basel (CH)**
Erfinder : **Lengsfeld, Hans, Dr.**
**Unterer Rebbergweg 96**
**CH-4153 Reinach (CH)**
Erfinder : **Weibel, Ernst Karl, Dr.**
**Vogtacherweg 3**
**CH-4133 Pratteln (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 129 748 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Das in FR-A-2379531 beschriebene Esterastin und dessen in FR-A-2426679 beschriebene Hydrierungsprodukt Tetrahydroesterastin sollen die Lipase hemmende Wirksamkeit haben und daher als Immunosuppresiva verwendbar sein. Das Esterastin wird aus Kulturen von Microorganismen der Spezies Streptomyces gewonnen. An Stelle der im Esterastin und Tetrahydroesterastin enthaltenen Acetamido- und Carbamoylmethylresten enthalten die Verbindungen der Formel I der vorliegenden Erfindung Formamido- und Isobutylreste.

Die vorliegende Erfindung betrifft Verbindungen der allgemeinen Formel

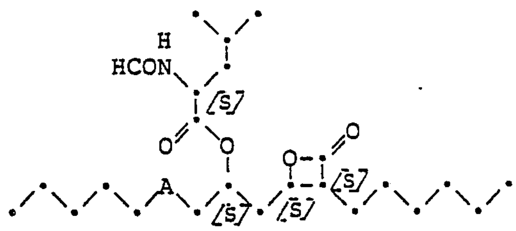

(I)

worin A die Gruppe

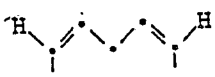

oder $-(CH_2)_5-$ bedeutet.

Die obige Formel I umfasst das (2S,3S,5S,7Z,10Z)-5-[(S)-2-Formamido-4-methyl-valeryloxy]-2-hexyl-3-hydroxy-7,10-hexadecadiensäurelacton der Formel

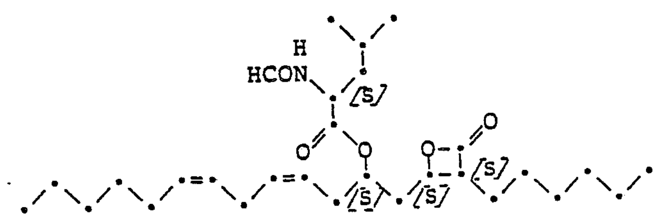

das nachstehend als Lipstatin bezeichnet wird, und das (2S,3S,5S)-5-[(S)-2-Formamido-4-methyl-valeryloxy]-2-hexyl-3-hydroxy-hexadecansäurelacton der Formel

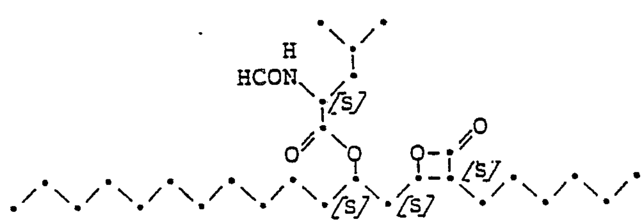

(Ib)

das nachstehend als Tetrahydrolipstatin bezeichnet wird.

Diese Verbindungen sind neu und besitzen wertvolle pharmakologische Eigenschaften. Sie hemmen insbesondere die Pankreaslipase und können bei der Bekämpfung oder Verhütung von Obesitas und Hyperlipämien verwendet werden.

Gegenstand der vorliegenden Erfindung sind die Verbindungen der obigen Formel I als solche und als pharmazeutische Wirkstoffe, die Herstellung dieser Verbindungen, Arzneimittel und industriell gefertigte Lebensmittel, enthaltend eine Verbindung der Formel I, deren Herstellung, sowie die Verwendung dieser Verbindungen bei der Bekämpfung oder Verhütung von Krankheiten.

Die Verdauung der mit der Nahrung aufgenommenen Fette (Triglyceride) erfolgt im Darm durch die Pankreaslipase. Die Pankreaslipase spaltet die primären Esterbindungen von Triglyceriden, wobei als Produkte freie Fettsäuren und 2-Monoglyceride entstehen. Diese Produkte können dann resorbiert und verwertet werden. Durch die Hemmung der Pankreaslipase wird die erwähnte Spaltung der Nahrungsfette und damit auch die Resorption und Verwertung dieser Stoffe teilweise verhindert ; die Triglyceride werden unverändert ausgeschieden.

Die Hemmung der Pankreaslipase durch die Verbindungen der Formel I kann experimentell gezeigt werden, indem man die bei der Spaltung von Triolein durch Schweinepankreaslipase freigesetzte Oelsäure titrimetrisch erfasst. Zu einer Emulsion, welche 1 mM Taurodeoxycholat, 9 mM Taurodeoleat, 0,1 mM Cholesterin, 1 mM Eilezithin, 15 mg/ml BSA, 2 mM Tris-HCl, 100 mM Natriumchlorid, 1 mM Calciumchlorid und das Substrat Triolein enthält, gibt man die in Aethanol oder Dimethylsulfoxid (10 % des Emulsionsvolumens) gelöste Verbindung der Formel I und startet die Reaktion durch Zugabe von 100 µl (175 U) Schweinepankreaslipase. Der pH wird während der Reaktion durch Zugabe von Natronlauge bei 8 gehalten. Aus dem während 10 Minuten ermittelten Verbrauch an Natronlauge wird die $IC_{50}$ berechnet. Die $IC_{50}$ ist diejenige Konzentration, bei der die Lipaseaktivität halbmaximal gehemmt wird. Die nachfolgende Tabelle I enthält die für die Verbindungen der Formel I ermittelten $IC_{50}$-Werte und Angaben über die akute Toxizität ($DL_{50}$ nach einmaliger oraler Verabreichung an Mäusen).

Tabelle I

| Testverbindung | $IC_{50}$ in µg/ml | $DL_{50}$ in mg/kg p.o. |
|---|---|---|
| Lipstatin | 0,07 | > 4000 |
| Tetrahydrolipstatin | 0,18 | - |

Die Hemmung der Resorption der mit der Nahrung aufgenommenen Fette, welche durch die Hemmung der Pankreaslipase bewirkt wird, kann in einem Doppelmarkierungs-Experiment an Mäusen gezeigt werden. Zu diesem Zweck verabreicht man den Versuchstieren eine Testmahlzeit, welche $^{3}H$-Triolein und $^{14}C$-Oelsäure enthält, und eine Verbindung der Formel I. Durch Messung der Radioaktivität ermittelt man dann die mit dem Kot ausgeschiedene Menge an $^{3}H$-Triolein und $^{14}C$-Oelsäure (in % der verabreichten Menge). Die in der nachfolgenden Tabelle II aufgeführten Resultate zeigen, dass im Vergleich zu unbehandelten Kontrolltieren die Ausscheidung von unverändertem Triglycerid stark erhöht und die Ausscheidung von Oelsäure weitgehend unverändert ist.

Tabelle II

| Testverbindung | Anzahl Versuchstiere | Dosis | Ausscheidung in % der verabreichten Menge | |
|---|---|---|---|---|
| | | | Triolein | Oelsäure |
| Kontrolle | 12 | - | 3,5 ± 0,3 | 10,1 ± 0,6 |
| Lipstatin | 6 | 40 mg/kg * | 56,8 ± 13 | 13,8 ± 5,6 |

* Die Versuche wurden mit einem Präparat durchgeführt, das etwa 10 % Lipstatin enthält. Die angegebene Dosis ist die verabreichte Menge an Lipstatin.

Die Verbindungen der Formel I können erfindungsgemäss hergestellt werden, indem man

a) zur Herstellung der Verbindung der Formel Ia einen diese Verbindung produzierenden Microorganismus der Spezies Streptomyces toxytricini in einem wässrigen Kulturmedium, das geeignete Kohlenstoff- und Stickstoffquellen und anorganische Salze enthält, aerob kultiviert und die produzierte Verbindung der Formel Ia aus der Kulturbrühe abtrennt, oder

b) zur Herstellung der Verbindung der Formel Ib die Verbindung der Formel Ia hydriert.

Aus Bodenproben von verschiedenen Orten konnten Streptomycetenstämme isoliert werden, welche Lipstatin, die Verbindung der Formel Ia, produzieren. Als Beispiel sei der aus einer in Mallorca, Spanien, gefundenen Bodenprobe isolierte Microorganismus genannt, der die Laborbezeichnung Streptomyces sp. 85-13 erhielt und durch CBS, Baarn (Niederlande), als Streptomyces toxytricini Preobrazhenskaya & Sveshnikova identifiziert worden ist (vgl. Bergey's Manual of Determinative Bacteriology, 8th Edition, Seite 811). Er erhielt hierauf die neue Bezeichnung Streptomyces toxytricini 85-13. Eine lyophilisierte Probe dieses Stammes wurde am 14. Juni 1983 bei der Agricultural Research Culture Collection, Peoria, Illinois, unter der Bezeichnung NRRL 15443 hinterlegt.

Es folgt die Beschreibung der Identifizierung von Streptomyces sp. 85-13 :

Medien

Die Zusammensetzung der verwendeten Medien ist in Int. J. Syst. Bacteriol. 1966, 16, 3 ; 313-321 beschrieben.

Nonomura-Diagramm

Nonomura benützte die Resultate des International Streptomyces Project (ISP) für die Klassifizierung der Streptomyceten-Spezies (J. Ferment. Technol. 1974, 52, 2).

Farben

Die Namen und Code-Nummern der Farben des Luftmycels stammen aus Tresner & Backus, « System of color wheels for streptomycete taxonomy ». Die Farben der Kolonierückseiten stammen aus H. Prauser's Selektion aus Baumann's « Farbtonkarte Atlas I ».

Methoden

Es wurde gemäss den ISP-Methoden verfahren (vgl. Int. J. Syst. Bacteriol. 1966, 16, 3 ; 313-340).

I. Agarkulturen nach 16 Tagen bei 28 °C (Doppelbestimmung)

a) Hafermehlagar

Wachstum : Sehr gut ; Kolonien : Dünn, sich ausbreitend ; Luftmycel : Samtartig, rosabraun (Light Brown 57) ; Kolonierückseite : Gelblich (Pr. Coo-3-m) mit breiten purpur-grauen Rändern (Pr. Oc-6-c) ; Lösliche Pigmente : Undeutlich.

b) Stärke-Salzagar

Wachstum : Gut ; Kolonien : Dünn, sich ausbreitend ; Luftmycel : Samtartig, rosabraun (Light Brown 57) mit weissem Sektor ; Kolonierückseite : Dunkel strohfarbig (Pr. Coo (Cr)5a), Ränder und andere Zonen rosa (Pr. Oc-5-b) mit einigen dunkelrotbraunen Punkten (Pr. 0-5-5(r)) ; Lösliche Pigmente : Undeutlich. Die stärkeabbauende Aktivität ist sehr ausgeprägt.

c) Glycerin-Asparaginagar

Wachstum : Gut ; Kolonien : Dünn, sich ausbreitend ; Luftmycel : Samtartig, hell rosabraun (R4ec : Grayish Yellowish Pink) ; Kolonierückseite : Orange (Pr. Oc-3-m/r) ; Lösliche Pigmente : blass rosabraun.

d) Hefe-Malzagar

Wachstum : Gut ; Kolonien : Dünn, gross ; Luftmycel : Samtartig, rötlichbraun (4ge : Light Grayish Reddish Brown 45) ; Kolonierückseite : Gelb (Pr. Coo-4-5) und dunkelbraun (Pr. Oc-5-r) ; Lösliche Pigmente : Sehr blasses Gelbbraun.

II. Agarkulturen nach 62 Tagen bei 28 °C (Doppelbestimmung)

a) Hafermehlagar

Wachstum : Gut ; Kolonien : Dünn, sich ausbreitend ; Luftmycel : Pulverig-samtartig, zimtfarbig (R-4ie : Light Brown (57)-Cork Tan) mit breitem, hellerem Rand (R. 5gc : Light Reddish Brown (4.2)-Peach Tan) ; Kolonierückseite : Gelblichbraun mit ockergelbem (Pr. Coo-3-a) Rand, leicht grau gegen das hellere Zentrum (Pr. Oc-4-r) ; Lösliche Pigmente : Helles Ockerbraun.

b) Stärke-Salzagar

Wie auf Hafermehlagar, aber mit stärker graubrauner Rückseite (Pr. Oc-6-c) und mit dunkelbraunen Flecken und Ringen an den Enden der Kreuzausstriche.

c) Glycerin-Asparaginagar

Wie auf Stärke-Salzagar, aber blasser, hell-beige (5ec : Grayish Yellowish Pink 32-Dusty Peads). Rückseite : Ockergelb (Pr. : Coo (= Cr)-4-b), heller im Zentrum ; keine löslichen Pigmente.

d) Hefe-Malzagar

Wachstum : Mässig ; Kolonien : Fast wie auf Hafermehlagar, aber mit sehr schmalem, blass grauem Rand ; Rückseite : Dunkelgelb (Pr. Coo-4-b) dunkelbraun in Randnähe ; Lösliche Pigmente : Undeutlich.

III. Melanoide Pigmente

Peptone-Hefeextrakt-Agar : Nach 24 Stunden negativ, nach 48 Stunden positiv ; Tyrosin-Agar : Nach 24 Stunden positiv, nach 48 Stunden positiv.

IV. Morphologie des sporulierenden Luftmycels

Sektion : Spira-Retinaculum Apertum. Verästelungsart : Sympodial. Spiralen oft irregulär, mit bis zu 5 Windungen und verschiedenen Durchmessern.

V. Verwertung von C-Quellen

Kein oder nur spurenweises Wachstum auf Arabinose, Xylose, Inosit, Mannit, Fructose, Rhamnose, Saccharose, Raffinose.

VI. Sporen

Oval bis zylindrisch-oval, manchmal unregelmässige Grösse, glatte Oberfläche. Sporenketten mit mehr als 10 Sporen.

VII. Nonomura-Diagramm

R(Gy) 100 SRA sm(±) (±) (±) -----

Für die Zwecke der vorliegenden Erfindung sind alle Streptomycetenstämme geeignet, die den Lipasehemmer Lipstatin produzieren, insbesondere Streptomyces toxytricini 85-13, NRRL 15443 und dessen Subkulturen, Mutanten und Varianten.

Die Kultivierung dieser Microorganismen zur Herstellung von Lipstatin kann nach verschiedenen Fermentationsmethoden durchgeführt werden. Sie kann beispielsweise im Schüttelkolben oder in 10 l- oder 200 l- und 1 000 l-Fermentern durchgeführt werden. Eine gewisse Menge Sporenmaterial oder Mycelium eines Lipstatin produzierenden Stammes wird in ein flüssiges Medium gebracht, das geeignete Kohlenstoff- und Stickstoffquellen und die für das Wachstum notwendigen Salze enthält, und bei einer Temperatur von 20-37 °C während 1-6 Tagen aerob bebrütet. Als Kohlenstoffquellen eignen sich beispielsweise Dextrin, Glucose, Stärke, Ribose und Glycerin. Geeignete Stickstoffquellen sind beispielsweise Hefeextrakt, Pepton oder Sojamehl. Als Salze kommen vorzugsweise Ammonium-, Magnesium- und Calciumsalze in Frage. Die Fermentation wird bei pH 6-8 durchgeführt.

Die Isolierung des Lipstatins erfolgt nach an sich bekannten und jedem Fachmann geläufigen Methoden und kann beispielsweise wie folgt durchgeführt werden :

Man zentrifugiert nach Beendigung der Fermentation die Gärbrühe, wonach 60-90 % der Aktivität in der Zellmasse und der Rest im Zentrifugat gefunden werden. Die Zellmasse kann dann mit einem niederen Alkohol, wie Methanol und Aethanol, behandelt und mit dem gleichen Lösungsmittel extrahiert werden. Das Zentrifugat kann mit einem geeigneten organischen Lösungsmittel, z. B. mit Methylenchlorid oder Essigester, extrahiert werden. Das aus den Extrakten gewonnene Material enthält das gewünschte Lipstatin und kann mittels chromatographischer Methoden angereichert und gereinigt werden. Geeignete Methoden sind beispielsweise die multiplikative Extraktion mit dem System Hexan/Methanol/Wasser (50 : 40 : 9), die Filtrationschromatographie über Kieselgel unter Eluieren mit Chloroform, die Säulenchromatographie an Kieselgel unter Eluieren mit Hexan, Essigester und Mischungen davon, die Chromatographie an unpolaren Trägermaterialien unter Eluieren mit polaren Lösungsmitteln, wie Methanol (Reversed-Phase-Chromatographie und die Hockdruck-Flüssigkeits-Chromatographie.

Die weiter unten folgenden Beispiele enthalten detaillierte Angaben betreffend die Kultivierung von Streptomyces toxytricini 85-13 und die Isolierung des Lipstatins.

Tetrahydrolipstatin, die Verbindung der Formel Ib, kann hergestellt werden, indem man Lipstatin in Gegenwart eines geeigneten Katalysators hydriert. Als Katalysatoren kommen beispielsweise Palladium/Kohle, Platinoxid, Palladium und dergleichen in Frage. Geeignete Lösungsmittel sind beispielsweise niedere Alkohole, wie Methanol und Aethanol. Man arbeitet vorzugsweise bei niedrigen Wasserstoffdrucken und bei Raumtemperatur.

Die Verbindungen der Formel I können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen ; je nach Beschaffenheit des Wirkstoffes sind jedoch bei Weichgelatinekapseln überhaupt keine Träger erforderlich. Zur Herstellung von Lösungen und Siurpen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I, ebenfalls Gegenstand der vorliegenden Erfindung, weiterhin auch ein Verfahren zur Herstellung solcher Arzneimittel, welches dadurch gekennzeichnet ist, dass man eine Verbindung der Formel I und gegebenenfalls einen oder mehrere andere therapeutisch wertvolle Stoffe in eine galenische Darreichungsform bringt. Wie eingangs erwähnt, können die Verbindungen der Formel I bei der Bekämpfung oder Verhütung von Krankheiten verwendet werden und zwar insbesondere bei der Bekämpfung oder Verhütung von Obesitas und Hyperlipämien. Die Dosierung kann innerhalb weiter Grenzen variieren und ist natürlich in jedem einzelen Fall den individuellen Gegebenheiten anzupassen. Im allgemeinen dürfte bei oraler Verabreichung eine Tagesdosis von etwa 0,1 mg bis 100 mg/kg Körpergewicht angemessen sein.

Die Verbindungen der Formel I können auch industriell gefertigen Lebensmitteln zugegeben werden, wobei insbesondere Fette, Oele, Butter, Margarine, Schokolade und andere Konfektartikel in Frage kommen. Solche industriell gefertigte Lebensmittel und deren Herstellung sind ebenfalls Gegenstand der vorliegenden Erfindung.

Die nachfolgenden Beispiele sollen die vorliegende Erfindung näher erläutern, ihren Umfang jedoch in keiner Weise beschränken. Sämtliche Temperaturen sind in Celsiusgraden angegeben.

## Beispiel 1

a) Fermentation

Ein Schüttelkolben mit dem Vorkulturmedium 391 wird mit Sporen von Streptomyces toxytricini 85-13 (oder vegetativem Mycel davon) beimpft und 72 Stunden bei 28 °C als Schüttelkultur aerob bebrütet. Etwa 2-5 Vol.-% dieser Kultur wird verwendet, um eine Fermentervorkultur von 10 l mit Vorkulturmedium 391 zu beimpfen. Man inkubiert während 3 Tagen bei 28°, wobei man mit 1 vvm belüftet und bei 400 RPM rührt. Diese 10 l-Vorkultur wird verwendet um einen 200-l-Produktionsfermenter mit dem Produktionsmedium N 7 zu beimpfen. Man fermentiert während 124 Stunden bei 28°, wobei man mit 1,0 vvm belüftet und bei 150 RPM rührt. Regelmässige Analysen zeigen nach 124 Stunden eine extrazelluläre lipasehemmende Aktivität von 53 $IC_{50}$/ml.

Das Vorkulturmedium 391 (pH 7,0) hat folgende Zusammensetzung : 3 % Maisstärke, 4 % Dextrin, 3 % Sojamehl, 0,2 % $(NH_4)_2SO_4$, 0,6 % $CaCO_3$ und 0,8 % Sojaöl. Der pH wurde auf 7 gestellt. Das Produktionsmedium N 7 (pH 7,0) hat folgende Zusammensetzung : 1 % Kartoffelstärke, 0,5 % Glucose, 1 % Ribose, 0,5 % Glycerin, 0,2 % Pepton, 2 % Sojamehl und 0,2 % $(NH_4)_2SO_4$.

b) Aufarbeitung

Man zentrifugiert die Gärbrühe mittels einer Röhrenzentrifuge, wobei man 175 l Kulturfiltrat und 12 kg Mycel erhält. Das Mycel wird verworfen, und das Kulturfiltrat wird während 10 Minuten auf 80° erhitzt, abgekühlt, nochmals zentrifugiert und bei 30° im Vakuum auf 50 l konzentriert. Man extrahiert dieses Konzentrat mit 50 l Hexan mittels eines kontinuierlich arbeitenden Extraktors, mischt die erhaltene Emulsion mit 50 l Hexan/Essigester (1 : 1) und trennt die organische Phase ab. Diese wird über Natriumsulfat getrocknet und eingedampft ; man erhält 199 g Rohextrakt I. Die wässrige Phase wird mit Wasser auf 100 l verdünnt und mit 100 l Essigester extrahiert. Man erhält nach dem Eindampfen der Essigesterlösung 49 g Rohextrakt II. Die wässrige Phase wird anschliessend ein weiteres Mal mit 100 l Essigester extrahiert, wobei nach dem Eindampfen 78 g Rohextrakt III erhalten werden.

c) Reinigung

Die Rohextrakte II und III werden in drei Portionen über je 1 kg Kieselgel 60 (0,040-0,063 mm Korngrösse) filtriert, wobei man mit Chloroform eluiert (Säule : 10 × 100 cm). Man erhält auf diese Weise 18,3 g angereichertes Material. 178 g dieser Substanz werden erneut unter Eluieren mit Chloroform über 1 kg Kieselgel filtriert. Man erhält dabei 5,29 g aktives Material. 802 mg dieser Substanz werden mittels Reversed-Phase-Chromatographie an einer im Handel erhältlichen Lobar-Fertigsäule (Lichoprep RP-8,

Grösse C) unter Eluieren mit Methanol gereinigt. Man erhält 158 mg (2S,3S,5S,7Z,10Z)-5-[(S)-2-Formamido-4-methyl-valeryloxy]-2-hexyl-3-hydroxy-7,10-hexadecadiensäurelacton (Lipstatin), das bei Raumtemperatur ein gelbliches Oel ist. Bei tiefen Temperaturen ist es wachsartig-kristallin.

Mikroanalyse (20 Stunden im Hochvakuum bei 50° getrocknet) :
Berechnet für $C_{29}H_{49}N_1O_5$ (491,713) : C 70,84, H 10,04, N 2,85
Gefunden : C 70,85, H 9,97, N 2,59
Optische Drehung : $[\alpha]_D^{20}$ = — 19,0° (c = 1, in Chloroform).
Massenspektrum (chemische Ionisation mit $NH_3$ als Reagenzgas) : Spitzen u. a. bei m/z 509 ($M + NH_4^+$) und 492 ($M + H^+$).
IR-Spektrum (Film) : Banden u. a. bei 3318, 3012, 2928, 2558, 2745, 1823, 1740, 1673, 1521, 1382, 1370, 1250, 1191 $cm^{-1}$.

Durch chemischen Abbau des Lipstatins und Vergleich der erhaltenen Bruchstücke mit bekannten Substanzen konnte die absolute Konfiguration festgelegt werden.

## Beispiel 2

a) Fermentation

Mit einer gemäss Beispiel 1 hergestellten Vorkultur von Streptomyces toxytricini 85-13 (Schüttelkolben und dann 10 l-Fermentation) wird eine 200 l-Fermentation mit Produktionsmedium N 16 beimpft. Das Produktionsmedium N 16 entspricht dem in Beispiel 1 verwendeten Produktionsmedium N 7, enthält jedoch zusätzlich 0,1 % Schweineschmalz. Die Fermentation wird während 120 Stunden wie in Beispiel 1 durchgeführt. Nach 120 Stunden beträgt die intrazelluläre lipasehemmende Aktivität 71 $IC_{50}$/ml, die extrazelluläre 4 $IC_{50}$/ml Gärbrühe.

b) Aufarbeitung

Nach Beendigung der Fermentation wird die Gärbrühe 10 Minuten auf 80° erhitzt, anschliessend abgekühlt und die Zellmasse mittels einer Röhrenzentrifuge aufgetrennt. Durch zweimaliges Zentrifugieren erhält man 11,4 kg Mycel ; das Kulturfiltrat wird verworfen. Das Mycel wird während 30 Minuten in 70 l Methanol verrührt, worauf man die erhaltene Suspension abnutscht. Der Filterkuchen wird nochmals mit 50 l Methanol verrührt und genutscht. Die vereinigten methanolischen Extrakte werden auf 1,8 l konzentriert. Dieses Konzentrat wird dreimal mit je 2 l Butylacetat extrahiert. Aus den vereinigten organischen Phasen erhält man nach dem Eindampfen 160 g Rohextrakt.

c) Reinigung

Dieser Rohextrakt wird durch multiplikative Extraktion mit dem System Hexan/Methanol/Wasser (5 : 4 : 0,9) gereinigt. Zuerst wird die aktive Substanz von der unteren Phase (uP) in die obere Phase (oP) transferiert. 160 g Rohextrakt werden in 4 l uP gelöst und im Ausrührgefäss mit 4 l oP gerührt. Nach der Abtrennung der oP wird die uP ein zweites Mal mit 4 l frischer oP extrahiert. Es bildet sich eine stabile Emulsion, welcher noch je 4 l uP und oP zugegeben werden, worauf eine gute Phasentrennung erzielt wird. Nach der Abtrennung der oP wird die uP noch zweimal mit 8 l frischer oP extrahiert. Die vereinigten oP ergeben nach dem, Eindampfen 90,3 g Extrakt. Die extrahierte uP wird verworfen. Nun wird die aktive Substanz von der oP in die uP transferiert. 90,3 g des obigen Extraktes werden in 4 l oP gelöst und mit 4 l uP extrahiert. Nach der Phasentrennung wird die oP noch dreimal mit frischer uP extrahiert. Die oP wird anschliessend verworfen. Die vereinigten uP werden auf 0,7 l wässrige Phase konzentriert, und diese wird achtmal mit insgesamt 0,2 l Essigester extrahiert. Nach dem Eindampfen erhält man 25,8 g Produkt. Die extrahierte wässrige Phase wird verworfen. Die weitere Reinigung dieses Materials erfolgt durch Filtration über 1 kg Kieselgel 60 (0,040-0,063 mm Korngrösse ; Säule 10 × 100 cm) unter Eluieren mit Chloroform. Man erhält 649 mg Produkt, das an einer Lobar-Fertigsäule (Lichoprep RP-8, Grösse C) unter Eluieren mit Methanol chromatographiert wird (Reversed-Phase-Chromatographie). Man erhält 204 mg Lipstatin, das gemäss Dünnschichtchromatogramm rein ist.

## Beispiel 3

Man löst 138 mg Lipstatin in 10 ml Aethanol, versetzt mit 60 mg 5-proz. Palladium/Kohle und rührt bei Raumtemperatur während 3 Stunden in einer Wasserstoffatmosphäre (Ballon). Anschliessend wird der Katalysator abzentrifugiert. Das Hydrierungsprodukt wird über eine kurze Kieselgelsäule (1 × 5 cm) mit Chloroform chromatographiert. Man erhält 112 mg (2S,3S,5S)-5-[(S)-2-Formamido-4-methyl-valeryloxy]-2-hexyl-3-hydroxy-hexadecansäurelacton (Tetrahydrolipstatin) als wachsartigen, schwach gelben Festkörper.
Optische Drehung : $[\alpha]_D^{20}$ = — 32,0° (c = 1, in Chloroform).

Massenspektrum (chemische Ionisation mit $NH_3$ als Reagenzgas) : Spitzen u. a. bei m/z 513 ($M + NH_4^+$) ; 496 ($M + H^+$) und 452 ($M + H^+ — CO_2$).

IR-Spektrum (Film) : Banden u. a. bei 3332, 2956, 2921, 2853, 1838, 1731, 1709, 1680, 1665, 1524, 1383, 1249 und 1200 $cm^{-1}$.

$^1H$-NMR-Spektrum (270 MHz, $CDCl_3$) : 0,89 (6H) ; 0,97 (6H) ; 1,15-1,5 (27H), 1,5-1,85 (6H) ; 1,9-2,25 (2H) ; 3,24 (1H) ; 4,32 (1H) ; 4,68 (1H) ; 5,03 (1H) ; 6,43 (1H) ; 8,07 und 8,21 (1H) ppm.

Beispiel 4

a) Fermentation

Eine 2 l-Schüttelkulturflasche mit Medium 391 wird mit Sporen einer Schrägagarkultur von Streptomyces toxytricini 85-13 beimpft und während 72 Stunden bei 28 °C aerob inkubiert. Danach wird die 2 l-Vorkultur in einen 50 l-Fermenter mit Produktionsmedium N 16 übergeführt und bei 28 °C während 77 Stunden mit 0,5 vvm Belüftung inkubiert. Diese 50 l-Vorkultur wird zur Beimpfung eines 1 000 l-Fermenters mit Medium N 16 verwendet. Diese Produktionsfermentation wird bei 28 °C und 0,5 vvm Belüftung während 91 Stunden durchgeführt, wobei ein Lipstatin-Titer von 73 $IC_{50}$/ml intrazellulär und 16 $IC_{50}$/ml extrazellulär erreicht wird. Die ganze Gärbrühe wird auf 2 °C gekühlt und zentrifugiert, wobei 41 kg feuchte Biomasse anfallen, die bei — 20 °C eingefroren werden.

b) Aufarbeitung

37 kg Mycel werden bei 4 °C aufgetaut und mit etwa 40 l Wasser in einem Mixer homogenisiert. Die erhaltene dünnflüssige Suspension wird mit 140 l Methanol versetzt und während 20 Minuten gerührt. Anschliessend wird über ein Filtertuch abgenutscht, worauf der Filterkuchen noch mit 140 l Methanol extrahiert wird. Die Methanolextrakte werden bei 30 °C auf etwa 22 l konzentriert. Das erhaltene Konzentrat wird mit Wasser auf 50 l verdünnt und im Ausrührgefäss dreimal mit je 50 l Hexan/Essigester (1 : 1) extrahiert. Bei der zweiten und dritten Extraktion erhält man Emulsionen, die durch Zugabe von etwa 1,4 kg bzw. 0,5 kg Kochsalz gebrochen werden können. Die vereinigten organischen Extrakte werden konzentriert, über Natriumsulfat getrocknet und bis zum öligen Rückstand eingedampft. Man erhält 428 g Rohextrakt.

c) Reinigung

Dieser Rohextrakt wird in vier Portionen über je 1 kg Kieselgel 60 (0,040-0,063 mm Korngrösse) filtriert, wobei man mit Chloroform eluiert (Säule : 10 × 100 cm). Man erhält 70 g angereichertes Präparat, das in zwei Portionen über je 1 kg Kieselgel 60 unter Eluieren mit Hexan/Essigester (Gradient von 9 : 1 bis 4 : 1) filtriert wird. Man erhält 4,2 g aktives Material, das man in vier Portionen mittels Reversed-Phase-Chromatographie an einer Lobar-Fertigsäule (Lichoprep RP-8, Grösse C) unter Eluieren mit Methanol reinigt. Man erhält 1,77 g Lipstatin.

Beispiel A

Herstellung von Weichgelatinekapseln folgender Zusammensetzung :

| | Menge pro Kapsel |
|---|---|
| Lipstatin | 50 mg |
| NEOBEE M-5 | 450 μl |

Die Lösung des Wirkstoffes in NEOBEE M-5 wird in Weichgelatinekapseln geeigneter Grösse abgefüllt.

**Patentansprüche** (für die Vertragsstaaten : BE, CH, DE, FR, GB, IT, LI, LU, NL, SE)

1. Eine Verbindung der allgemeinen Formel

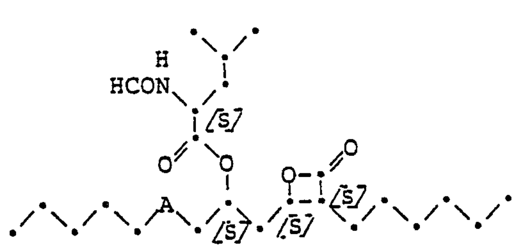

(I)

worin A die Gruppe

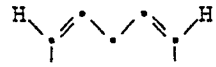

oder $-(CH_2)_5-$ bedeutet.

2. (2S,3S,5S,7Z,10Z)-5-[(S)-2-Formamido-4-methyl-valeryloxy]-2-hexyl-3-hydroxy-7,10-hexadecadiensäurelacton.

3. (2S,3S,5S)-5-[(S)-2-Formamido-4-methyl-valeryloxy]-2-hexyl-3-hydroxy-hexadecansäurelacton.

4. Eine Verbindung gemäss Anspruch 1, 2 oder 3 zur Anwendung als therapeutischer Wirkstoff.

5. Eine Verbindung gemäss Anspruch 1, 2 oder 3 zur Anwendung als ein die Pankreaslipase hemmender Wirkstoff.

6. Verfahren zur Herstellung einer Verbindung gemäss Anspruch 1, 2 oder 3, dadurch gekennzeichnet, dass man

a) zur Herstellung von (2S,3S,5S,7Z,10Z)-5-[(S)-2-Formamido-4-methyl-valeryloxy]-2-hexyl-3-hydroxy-7,10-hexadecadiensäurelacton der Formel

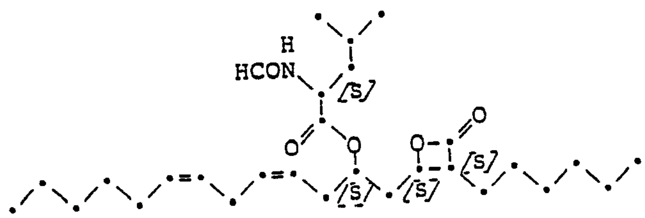

(Ia)

einen die Verbindung der Formel Ia produzierenden Microorganismus der Spezies Streptomyces toxytricini in einem wässrigen Kulturmedium, das geeignete Kohlenstoff- und Stickstoffquellen und anorganische Salze enthält, aerob kultiviert und die produzierte Verbindung der Formel Ia aus der Kulturbrühe abtrennt, oder

b) zur Herstellung von (2S,3S,5S)-5-[(S)-2-Formamido-4-methyl-valeryloxy]-2-hexyl-3-hydroxy-hexadecansäurelacton der Formel

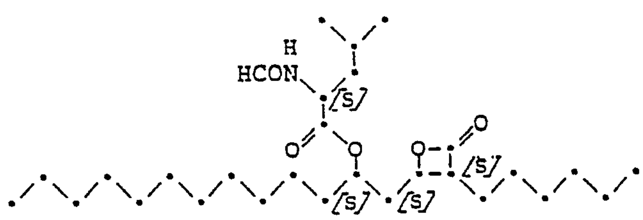

(Ib)

die Verbindung der Formel Ia hydriert.

7. Verfahren gemäss Anspruch 6, dadurch gekennzeichnet, dass man als Microorganismus Streptomyces toxytricini NRRL 15443 oder die Verbindung der Formel Ia produzierende Subkulturen, Varianten oder Mutanten davon verwendet.

8. Arzneimittel, enthaltend eine Verbindung gemäss Anspruch 1, 2 oder 3 und ein therapeutisch inertes Trägermaterial.

9. Arzneimittel gemäss Anspruch 8, welche die Pankreaslipase hemmen.

10. Industriell gefertige Lebensmittel, enthaltend eine Verbindung gemäss Anspruch 1, 2 oder 3.

**Patentansprüche** (für den Vertragsstaat AT)

1. Eine Verbindung der allgemeinen Formel

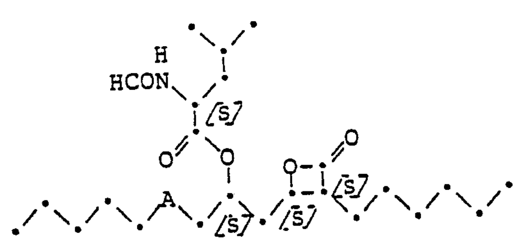

(I)

worin A die Gruppe

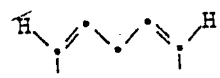

oder $-(CH_2)_5-$ bedeutet.

2. (2S,3S,5S,7Z,10Z)-5-[(S)-2-Formamido-4-methyl-valeryloxy]-2-hexyl-3-hydroxy-7,10-hexadecadiensäurelacton.

3. (2S,3S,5S)-5-[(S)-2-Formamido-4-methyl-valeryloxy]-2-hexyl-3-hydroxy-hexadecansäurelacton.

4. Eine Verbindung gemäss Anspruch 1, 2 oder 3 zur Anwendung als therapeutischer Wirkstoff.

5. Eine Verbindung gemäss Anspruch 1, 2 oder 3 zur Anwendung als ein die Pankreaslipase hemmender Wirkstoff.

6. Verfahren zur Herstellung einer Verbindung der allgemeinen Formel

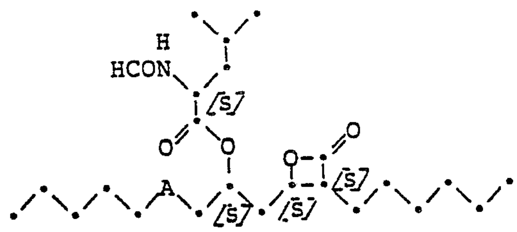

(I)

worin A die Gruppe

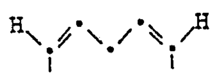

oder $-(CH_2)_5-$ bedeutet,
dadurch gekennzeichnet, dass man

a) zur Herstellung von (2S,3S,5S,7Z,10Z)-5-[(S)-2-Formamido-4-methyl-valeryloxy]-2-hexyl-3-hydroxy-7,10-hexadecadiensäurelacton der Formel

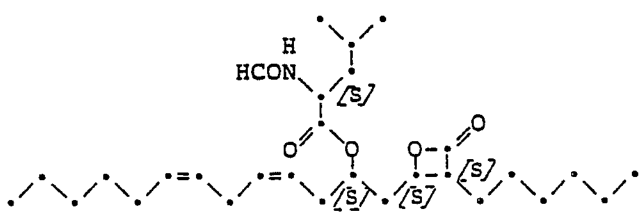

(Ia)

einen die Verbindung der Formel Ia produzierenden Microorganismus der Spezies Streptomyces toxytricini in einem wässrigen Kulturmedium, das geeignete Kohlenstoff- und Stickstoffquellen und anorganische Salze enthält, aerob kultiviert und die produzierte Verbindung der Formel Ia aus der Kulturbrühe abtrennt, oder

b) zur Herstellung von (2S,3S,5S)-5-[(S)-2-Formamido-4-methyl-valeryloxy]-2-hexyl-3-hydroxy-hexadecansäurelacton der Formel

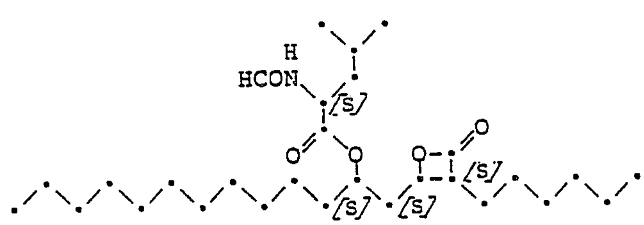

(Ib)

die Verbindung der Formel Ia hydriert.

7. Verfahren gemäss Anspruch 6, dadurch gekennzeichnet, dass man als Microorganismus Streptomyces toxytricini NRRL 15443 oder die Verbindung der Formel Ia produzierende Subkulturen, Varianten oder Mutanten davon verwendet.

8. Verfahren nach Anspruch 6, dadurch gekennzeichnet, dass man (2S,3S,5S,7Z,10Z)-5-[(S)-2-Formamido-4-methyl-valeryloxy]-2-hexyl-3-hydroxy-7,10-hexadecadiensäurelacton herstellt.

9. Verfahren nach Anspruch 6, dadurch gekennzeichnet, dass man (2S,3S,5S)-5-[(S)-2-Formamido-4-methyl-valeryloxy]-2-hexyl-3-hydroxy-hexadecansäurelacton herstellt.

10. Arzneimittel ; enthaltend eine Verbindung gemäss Anspruch 1, 2 oder 3 und ein therapeutisch inertes Trägermaterial.

11. Arzneimittel gemäss Anspruch 10, welche die Pankreaslipase hemmen.

12. Industriell gefertige Lebensmittel, enthaltend eine Verbindung gemäss Anspruch 1, 2 oder 3.

**Claims** (for the Contracting States : BE, CH, DE, FR, GB, IT, LI, LU, NE, SE)

1. A compound of the general formula

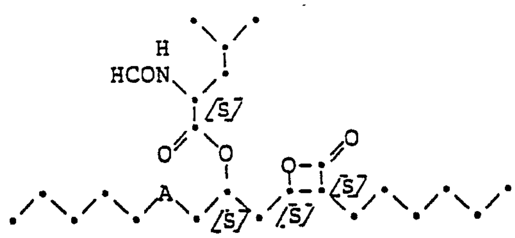

(I)

wherein A signifies the group

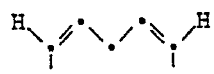

or $-(CH_2)_5-$.

2. (2S,3S,5S,7Z,10Z)-5-[(S)-2-Formamido-4-methyl-valeryloxy]-2-hexyl-3-hydroxy-7,10-hexadecadienoic acid lactone.

3. (2S,3S,5S)-5-[(S)-2-Formamido-4-methyl-valeryloxy]-2-hexyl-3-hydroxy-hexadecanoic acid lactone.

4. A compound in accordance with claim 1, 2 or 3 for use as a therapeutically active substance.

5. A compound in accordance with claim 1, 2 or 3 for use as an active substance which inhibits pancreas lipase.

6. A process for the manufacture of a compound in accordance with claim 1, 2 or 3, characterized by

a) for the manufacture of (2S,3S,5S,7Z,10Z)-5-[(S)-2-formamido-4-methyl-valeryloxy]-2-hexyl-3-hydroxy-7,10-hexadecadienoic acid lactone of the formula

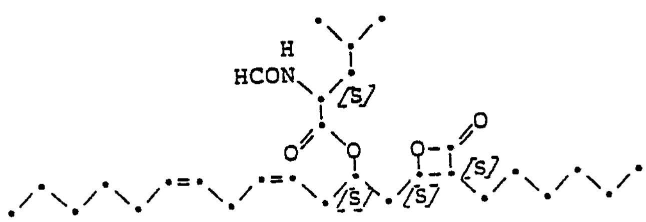

(Ia)

aerobically cultivating a microorganism of the species Streptomyces toxytricini which produces the compound of formula Ia in an aqueous culture medium which contains suitable carbon and nitrogen sources and inorganic salts and separating the compound of formula Ia produced from the culture broth, or

b) for the manufacture of (2S,3S,5S)-5-[(S)-2-formamido-4-methyl-valeryloxy]-2-hexyl-3-hydroxy-hexadecanoic acid lactone of the formula

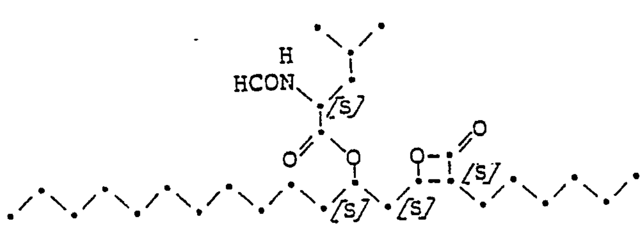

(Ib)

hydrogenating the compound of formula Ia.

7. A process in accordance with claim 6, characterized in that Streptomyces toxytricini NRRL 15443 or a subculture, variat or mutant thereof which produces the compound of formula Ia is used as the microorganism.

8. A medicament containing a compound in accordance with claim 1, 2 or 3 and a therapeutically inert carrier material.

9. A medicament in accordance with claim 8 which inhibits pancreas lipase.

10. A industrially-produced foodstuff containing a compound in accordance with claim 1, 2 or 3.

**Claims** (for the Contracting State AT)

1. A compound of the general formula

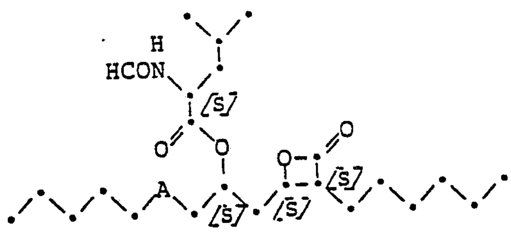

(I)

wherein A signifies the group

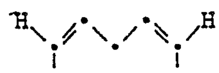

or $-(CH_2)_5-$.

2. (2S,3S,5S,7Z,10Z)-5-[(S)-2-Formamido-4-methyl-valeryloxy]-2-hexyl-3-hydroxy-7,10-hexadecadienoic acid lactone.

3. (2S,3S,5S)-5-[(S)-2-Formamido-4-methyl-valeryloxy]-2-hexyl-3-hydroxy-hexadecanoic acid lactone.

4. A compound in accordance with claim 1, 2 or 3 for use as a therapeutically active substance.

5. A compound in accordance with claim 1, 2 or 3 for use as an active substance which inhibits pancreas lipase.

6. A process for the manufacture of a compound of the general formula

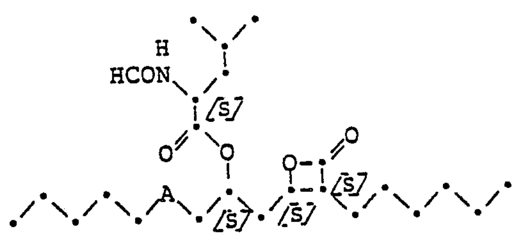

(I)

wherein A signifies the group

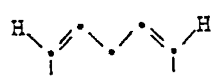

or $-(CH_2)_5-$,
characterized by

a) for the manufacture of (2S,3S,5S,7Z,10Z)-5-[(S)-2-formamido-4-methyl-valeryloxy]-2-hexyl-3-hydroxy-7,10-hexadecadienoic acid lactone of the formula

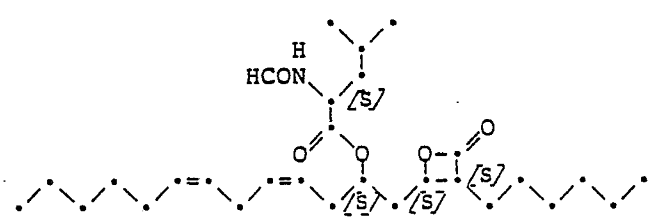

(Ia)

aerobically cultivating a microorganism of the species Streptomyces toxytricini which produces the compound of formula Ia in an aqueous culture medium which contains suitable carbon and nitrogen sources and inorganic salts and separating the compound of formula Ia produced from the culture broth, or

b) for the manufacture of (2S,3S,5S)-5-[(S)-2-formamido-4-methyl-valeryloxy]-2-hexyl-3-hydroxy-hexadecanoic acid lactone of the formula

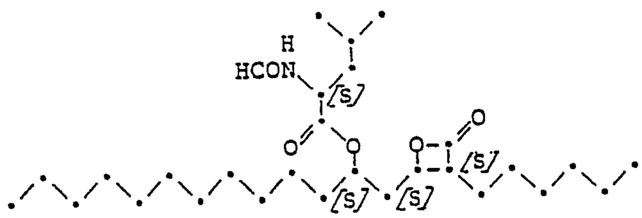

(Ib)

hydrogenating the compound of formula Ia.

7. A process in accordance with claim 6, characterized in that Streptomyces toxytricini NRRL 15443 or a subculture, variant or mutant thereof which produces the compound of formula Ia is used as the microorganism.

8. A process according to claim 6, characterized in that (2S,3S,5S,7Z,10Z)-5-[(S)-2-formamido-4-methyl-valeryloxy]-2-hexyl-3-hydroxy-7,10-hexadecadienoic acid lactone is manufactured.

9. A process according to claim 6, characterized in that (2S,3S,5S)-5-[(S)-2-formamido-4-methyl-valeryloxy]-2-hexyl-3-hydroxy-hexadecanoic acid lactone is manufactured.

10. A medicament containing a compound in accordance with claim 1, 2 or 3 and a therapeutically inert carrier material.

11. A medicament in accordance with claim 10 which inhibits pancreas lipase.

12. An industrially-produced foodstuff containing a compound in accordance with claim 1, 2 or 3.

**Revendications** (pour les Etats contractants : BE, CH, DE, FR, GB, IT, LI, LU, NL, SE)

1. Composé de formule générale

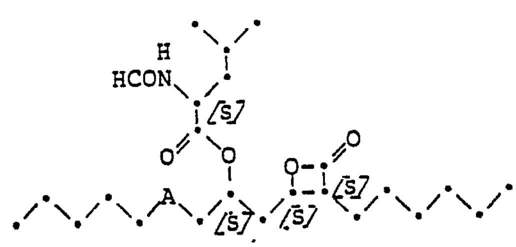

(I)

où A représente le groupe

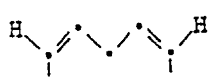

ou $-(CH_2)_5-$

2. Lactone de l'acide (2S,3S,5S,7Z,10Z)-5-[(S)-2-formamido-4-méthyl-valéryloxy]-2-hexyl-3-hydroxy-7,10-hexadécadiénoïque.

3. Lactone de l'acide (2S,3S,5S)-5-[(S)-2-formamido-4-méthyl-valéryloxy]-2-hexyl-3-hydroxy-hexadécanoïque.

4. Composé selon la revendication 1, 2 ou 3 aux fins d'application comme substance active thérapeutique.

5. Composé selon la revendication 1, 2 ou 3 aux fins d'application comme substance active inhibant la lipase pancréatique.

6. Procédé de préparation d'un composé selon la revendication 1, 2 ou 3, caractérisé en ce que

a) pour préparer la lactone de l'acide (2S,3S,5S,7Z,10Z)-5-[(S)-2-formamido-4-méthyl-valéryloxy]-2-hexyl-3-hydroxy-7,10-hexadécadiénoïque de formule

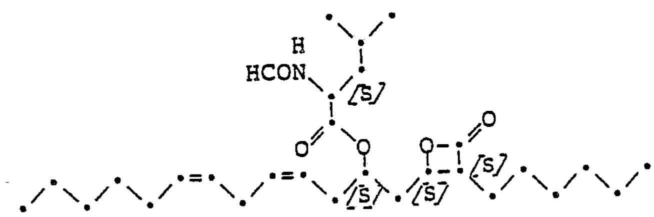

(Ia)

on cultive de façon aérobie un microorganisme de l'espèce Streptomyces toxytricini produisant le composé de formule la dans un milieu de culture aqueux qui contient les sources de carbone et d'azote appropriées et des sels inorganiques, et on sépare de la bouillie de culture le composé de formule la produit, ou

b) pour préparer la lactone de l'acide (2S,3S,5S)-5-[(S)-2-formamido-4-méthyl-valéryloxy]-2-hexyl-3-hydroxy-hexadécanoïque de formule

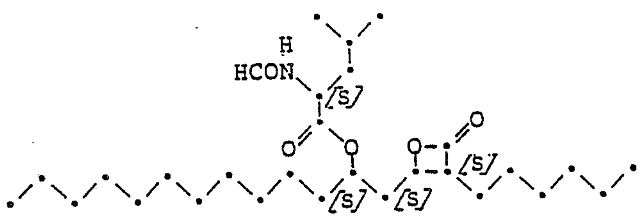

(Ib)

on hydrogène le composé de formule la.

7. Procédé selon la revendication 6, caractérisé en ce qu'on utilise comme microorganisme Streptomyces toxytricini NRRL 15443 ou ses sous-cultures, variantes ou mutants produisant le composé de formule la.

8. Médicament contenant un composé selon la revendication 1, 2 ou 3 et un support thérapeutiquement inerte.

9. Médicaments selon la revendication 8 qui inhibent la lipase pancréatique.

10. Aliments préparés industriellement, contenant un composé selon la revendication 1, 2 ou 3.

**Revendication** (pour l'Etat contractant AT)

1. Composé de formule générale

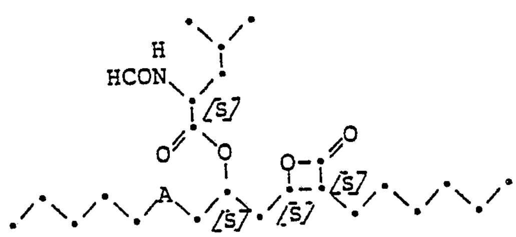

(I)

où A représente le groupe

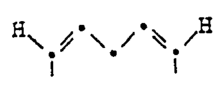

ou $-(CH_2)_5-$.

2. Lactone de l'acide (2S,3S,5S,7Z,10Z)-5-[(S)-2-formamido-4-méthyl-valéryloxy]-2-hexyl-3-hydroxy-7,10-hexadécadiénoïque.

3. Lactone de l'acide (2S,3S,5S)-5-[(S)-2-formamido-4-méthyl-valéryloxy]-2-hexyl-3-hydroxy-hexadécanoïque.

4. Composé selon la revendication 1, 2 ou 3 aux fins d'application comme substance active thérapeutique.

5. Composé selon la revendication 1, 2 ou 3 aux fins d'application comme substance active inhibant la lipase pancréatique.

6. Procédé de préparation d'un composé de formule générale

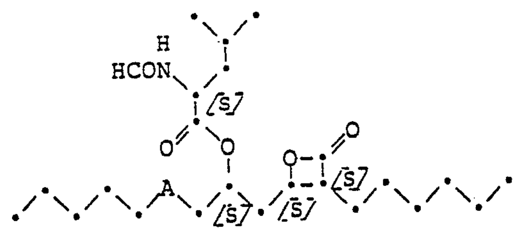

(I)

où A représente le groupe

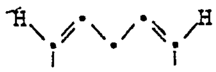

ou $-(CH_2)_5-$,
caractérisé en ce que

a) pour préparer la lactone de l'acide (2S,3S,5S,7Z,10Z)-5-[(S)-2-formamido-4-méthyl-valéryloxy]-2-hexyl-3-hydroxy-7,10-hexadécadiénoïque de formule

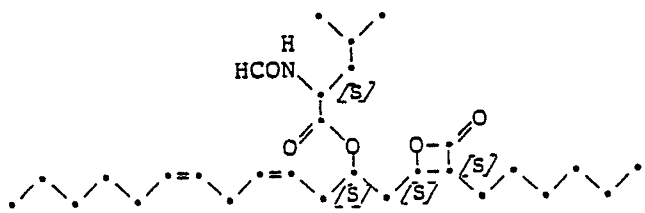

(Ia)

on cultive de façon aérobie un microorganisme de l'espèce Streptomyces toxytricini dans un milieu de culture aqueux qui contient les sources de carbone et d'azote appropriées et des sels inorganiques, et on sépare de la bouillie de culture le composé de formule Ia produit, ou

b) pour préparer la lactone de l'acide (2S,3S,5S)-5-[(S)-2-formamido-4-méthyl-valéryloxy]-2-hexyl-3-hydroxy-hexadécanoïque de formule

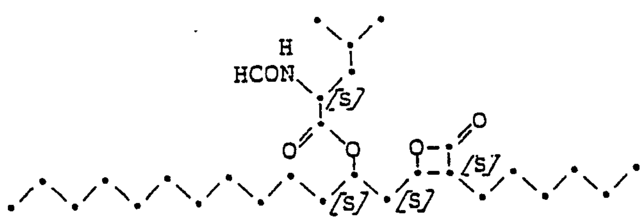

(Ib)

on hydrogène le composé de formule Ia.

7. Procédé selon la revendication 6, caractérisé en ce qu'on utilise comme microorganisme Streptomyces toxytricini NRRL 15443 ou ses sous-cultures, variantes ou mutants produisant le composé de formule Ia.

8. Procédé selon la revendication 6, caractérisé en ce qu'on prépare la lactone de l'acide (2S,3S,5S,7Z,10Z)-5-[(S)-2-formamido-4-méthyl-valéryloxy]-2-hexyl-3-hydroxy-7,10-hexadécadiénoïque.

9. Procédé selon la revendication 6, caractérisé en ce qu'on prépare la lactone de l'acide (2S,3S,5S)-5-[(S)-2-formamido-4-méthyl-valéryloxy]-2-hexyl-3-hydroxy-hexadécadiénoïque.

10. Médicament contenant un composé selon la revendication 1, 2 ou 3 et un support thérapeutiquement inerte.

11. Médicaments selon la revendication 10 qui inhibent la lipase pancréatique.

12. Aliments préparés industriellement, contenant un composé selon la revendication 1, 2 ou 3.